# EUROPEAN PATENT SPECIFICATION

(11) **EP 3 487 519 B1**
(45) Date of publication and mention of the grant of the patent: **07.06.2023**
(21) Application number: 17746021.9
(22) Date of filing: 20.07.2017
(51) Int. Cl.: A61K 35/30, A61K 33/00, A61K 33/06, A61K 33/14, A61K 33/42, A61K 38/17, A61K 31/41, A61K 31/4178, A61K 31/437, A61K 31/55, A61K 31/5517, A61K 45/06, A61P 27/02, A61P 27/06

(54) **COMPOUNDS FOR USE IN METHODS FOR TREATING GLAUCOMA AND RETINAL DISEASES**
VERBINDUNGEN ZUR ANWENDUNG IN VERFAHREN ZUR BEHANDLUNG VON GLAUKOM UND NETZHAUTERKRANKUNGEN
COMPOSÉS POUR LA MISE EN OEUVRE DE PROCÉDÉS DE TRAITEMENT DU GLAUCOME ET DES MALADIES DE LA RÉTINE

(30) Priority: 20.07.2016 EP 16180333
(43) Date of publication of application: 29.05.2019
(73) Proprietor: P&X Medical NV, 8020 Oostkamp (BE)
(72) Inventor: WOSTYN, Peter, 8670 Oostduinkerke (BE); DE DEYN, Peter, Paul, 2020 Antwerpen (BE); KILLER, Hanspeter, Esriel, 5034 Suhr (CH)
(74) Representative: De Clercq & Partners
(86) International application number: PCT/EP2017/068296
(87) International publication number: WO 2018/015467

(56) References cited:
- WO-A1-2016/059162
- US-A1- 2007 203 211
- US-A1- 2007 225 225
- US-A1- 2007 281 978
- US-A1- 2016 000 945
- US-B1- 6 268 359
- US-B2- 8 785 491
- SHAH G B ET AL: "OCULOHYPOTENSIVE EFFECT OF ANGIOTENSIN-CONVERTING ENZYME INHIBITORSIN ACUTE AND CHRONIC MODELS OF GLAUCOMA", JOURNAL OF CARDIOVASCULAR PHARMACOLOGY, RAVEN PRESS, NEW YORK, NY, vol. 36, no. 2, 1 January 2000 (2000-01-01), pages 169-175, XP000980137, ISSN: 0160-2446, DOI: 10.1097/00005344-200008000-00005
- Anonymous: "Glaucoma - Eye Disorders - Merck Manuals Consumer Version", Merck Manual , 25 December 2015 (2015-12-25), XP055335779, Retrieved from the Internet: URL:http://web.archive.org/web/20151225222 232/https://www.merckmanuals.com/home/eye- disorders/glaucoma/glaucoma [retrieved on 2017-01-16]
- WOSTYN P ET AL: "A new glaucoma hypothesis: A role of glymphatic system dysfunction", FLUIDS AND BARRIERS OF THE CNS, vol. 12, no. 16, 20 June 2015 (2015-06-20) , pages 1-6, XP008182881, ISSN: 2045-8118, DOI: 10.1186/S12987-015-0012-Z [retrieved on 2015-06-29]
- DENNISTON A K ET AL: "Paravascular pathways in the eye: Is there an ocular glymphatic system ?", INVESTIGATIVE OPHTHALMOLOGY & VISUAL SCIENCE - IOVS, ASSOCIATION FOR RESEARCH IN VISION AND OPHTHALMOLOGY, US, vol. 56, no. 6, 1 June 2015 (2015-06-01), pages 3955-3956, XP008182880, ISSN: 0146-0404, DOI: 10.1167/IOVS.15-17243
- HU PING ET AL: "Evidence for a Glymphatic System in Human, Primate, Rat and Mouse Retina", IOVS, vol. 57, no. 12, September 2016 (2016-09), XP009500471, & ANNUAL MEETING OF THE ASSOCIATION-FOR-RESEARCH-IN-VISION-AND-OPH THALMOLOGY (ARVO); SEATTLE, WA, USA; MAY 01 -05, 2016
- LOEFFLER JANA ET AL: "Retinal Amyloid Precursor Protein (APP) processing and amyloid-beta (A beta) transport in an Alzheimer's disease (AD) mouse model", IOVS, vol. 57, no. 12, September 2016 (2016-09), page 2270, XP009500475, & ANNUAL MEETING OF THE ASSOCIATION-FOR-RESEARCH-IN-VISION-AND-OPH THALMOLOGY (ARVO); SEATTLE, WA, USA; MAY 01 -05, 2016

## Description

### FIELD OF THE INVENTION

The present application relates generally to the field of retinal diseases and compositions for use in the treatment thereof. More particularly, the application provides compositions for use in treating and/or preventing retinal diseases selected from age-related macular degeneration, retinal vasculitis and retinal infective processes and commotio retinae, diabetic retinopathy, hereditary retinal dystrophies, ischemic insult of retinal neurons and macular edema, by facilitating glymphatic clearance in the optic nerve and/or retina.

### BACKGROUND

Glaucoma is one of the leading causes of irreversible blindness. The most common type of glaucoma is primary open-angle glaucoma (POAG), which is a progressive optic neuropathy with characteristic structural changes in the optic nerve head and corresponding visual field defects. The optic nerve is a white matter tract of the central nervous system (CNS) that extends into the orbit where it is surrounded by cerebrospinal fluid (CSF) in the subarachnoid space (SAS). It is ensheathed in the meninges throughout its intraorbital and intracanalicular course. The meningeal sheath of the optic nerve has the same lamellar structure as the meninges of the brain. It consists of the pia mater, the arachnoid mater and the dura mater. In the glaucomatous optic nerve, cupping of the optic disc reflects a loss of retinal ganglion cell (RGC) axons and a posterior bowing of the lamina cribrosa (forming the anatomic floor of the optic nerve head), accompanied by extensive remodeling of the optic nerve head.

Additionally, age-related macular degeneration (AMD) is a chronic neurodegenerative condition that affects retinal neurons and leads to progressive and irreversible loss of the vision. Age-related macular degeneration, a leading cause of severe vision impairment in people aged over 60 years, affects the macular region of the retina which is critical for the high resolution central vision (Gupta V, et al. Cell Mol Life Sci 2016). It is a progressive retinal degenerative disease influenced by both environmental and genetic factors (Ding JD, et al. Proc Natl Acad Sci U S A 2011;108:E279-87). Although the presence of a few small hard drusen is a normal, nonvision-impairing part of aging, the deposition of large diffuse drusen in the macula adversely impacts vision and is indicative of early AMD. As AMD progresses to late-stage disease, it is categorized as either dry (geographic atrophy with photoreceptor loss and extensive atrophy of the retinal pigmented epithelium (RPE)) or wet (exudative with subsequent choroidal neovascularization (CNV)). Currently, there are no effective treatments for early AMD, and treatments for late-stage disease are limited to photodynamic therapy, macular translocation, and antivascular endothelial growth factor drugs. The use of angiotensin II receptor antagonists such as valsartan an losartan is suggested in US2007/203211.

The strongest known risk factors are advanced age and cigarette smoking, with additional risk conferred by body mass index and diets high in fat. The last decade has also yielded strong evidence that genotype, especially for genes involved in inflammation and the innate immune system, influences AMD risk and progression. Genes implicated as risk factors include complement factor H (CFH), complement factor B, complement C3, apolipoprotein E (APOE), toll-like receptor 4, LOC387715/ARMS2, HTRA1, ABCA4, and fibulin 5. Additional support for a role for chronic local inflammation in AMD comes from the discovery that protein components of drusen include activated components of the complement system (e.g., C3b and C5b-9), molecules involved in the acute-phase response to inflammation (e.g., amyloid P component), and proteins that modulate the immune response (e.g., CFH, vitronectin, clusterin/apolipoprotein J, apolipoprotein E (apoE), and amyloid-β (Aβ). Several studies found that Aβ may play an inportant role in the pathogenesis of age-related macular degeneration (AMD). In human AMD, Aβ deposition is associated with drusen, where it accumulates and colocalizes with activated complement components. Aβ peptide has been detected in sub-retinal pigmented epithelium (RPE) basal deposits and neovascular lesions in a murine model of AMD (Ding JD, et al. Vision Res 2008;48:339-45; Malek G, et al. Proc Natl Acad Sci U S A 2005;102:11900-5). In this model, aged human APOE4-targeted replacement mice (APOE4 mice) fed a high-fat, cholesterol-enriched (HFC) diet (APOE4-HFC mice) exhibit morphologic hallmarks observed in both dry and wet AMD. These hallmarks include thick diffuse sub-RPE basal deposits, lipid- and protein-containing focal drusen-like deposits, thickening of Bruch's membrane, patchy regions of RPE atrophy opposed to areas of photoreceptor degeneration, and choroidal neovascularization (CNV).

The brain and spinal cord are encased within the cranium and vertebral column inside a thin membrane known as the arachnoid. The volume of the intracranial space is on average about 1700 ml including volumes of approximately 1400 ml of brain, approximately 150 ml of intracranial blood; and approximately 150 ml of CSF. CSF is mainly produced (i.e. about 80% of the total volume) by choroid plexuses within the brain ventricles, from where it flows into interconnecting chambers, namely, the cisterns and the SASs, including the SAS of the optic nerve. The sources of the remainder of CSF are the vasculature of the subependymal regions, and the pia mater. The total volume of CSF is renewed several times per day, so that about 500 ml are produced every 24 hours. CSF is absorbed through the arachnoid villi, located principally over the superior surfaces of the cerebral hemispheres. Some villi also exist at the base of the brain and along the roots of the spinal nerves. The absorptive processes include bulk transport of large molecules and as well as diffusion across porous membranes of small molecules.

According to the traditional understanding of CSF physiology, CSF is mainly produced by choroid plexuses within the brain ventricles, from where it flows into interconnecting chambers, namely, the cisterns and the subarachnoid spaces (SASs), including the SAS of the optic nerve.

Raised intraocular pressure is recognized as one of the most important risk factors for POAG and lowering it remains the only current therapeutic approach for slowing optic nerve damage and visual field progression in glaucoma patients. Known glaucoma therapies include medicines (e.g., prostaglandin analogues, beta-blockers, carbonic anhydrase inhibitors, and alpha-agonists), laser surgery (e.g., laser trabeculoplasty), and incisional surgery (e.g., trabeculectomy, deep sclerectomy, viscocanalostomy, and glaucoma drainage implants).

Therapy typically starts from the least invasive options, which usually involve the administration of medication. However, the administration of medication often fails for various reasons. Indeed, medicaments for the treatment of POAG typically lower the IOP by at most about 25% to 30%, which can be insufficient. Some glaucoma patients show disease progression despite of the administration of medicaments. Moreover, topical medications for glaucoma can cause side effects such as precipitation of asthma, bradycardia, impotence, and decreased exercise tolerance. There is also a significant problem in compliance with glaucoma medications due to frequent dosing regimens. Incisional surgery is usually required when (topical) glaucoma medication and/or laser surgery fail. However, current incisional surgery techniques for treating glaucoma can lead to various complications including but not limited to choroidal effusion, hypotony maculopathy, suprachoroidal haemorrhage, and bleb infections.

Accordingly, there is a need for an alternative glaucoma treatment which mitigates at least one of the above problems.

The glymphatic system was first described by Iliff et al. in 2012 (Iliff et al., Sci Transl Med. 2012;4:147ra111). The authors defined for the first time a brain-wide network of paravascular channels, which they termed the 'glymphatic pathway', along which CSF moves into and through the brain parenchyma, facilitating the exchange of CSF and ISF and the clearance of interstitial solutes from the brain. This was based upon in vivo two-photon microscopy and ex vivo fluorescence imaging of intracisternally infused CSF tracers in mice. Their findings suggested the existence of a brain-wide network of paravascular channels along which a large proportion of subarachnoid CSF recirculates through the brain parenchyma, facilitating the clearance of interstitial solutes, including amyloid-β, from the brain. This anatomical pathway consists of 3 elements: a para-arterial 4

CSF influx route, a para-venous ISF clearance route, and a trans-parenchymal pathway that is dependent upon astroglial water transport via the astrocytic aquaporin-4 water channel.

### SUMMARY OF THE INVENTION

The references to methods of treatment in the summary and detailed description of the invention in this description are to be interpreted as references to the compounds, pharmaceutical compositions and medicaments of the present invention for use in a method for treatment of the human (or animal) body by therapy. The present invention provides a composition comprising CSF or a CSF-like composition for use in the prevention and/or treatment of a retinal disease; - wherein the CSF-like composition is artificial CSF (aCSF), wherein aCSF comprises sodium ions at a concentration of 140-190 mM, potassium ions at a concentration of 2.5-4.5 mM, calcium ions at a concentration of 1-1.5 mM, magnesium ions at a concentration of 0.5-1.5 mM, phosphor ions at a concentration of 0.5-1.5 mM, chloride ions a concentration of 100-200 mM; - wherein said retinal disease is a disease primarily affecting the retina; and - wherein said prevention or treatment of retinal diseases is ensured by infusion into the intrathecal space, subarachnoid space or cerebral ventricles and - wherein said retinal disease is selected from age-related macular degeneration, retinal vasculitis and retinal infective processes, commotio retinae, diabetic retinopathy, hereditary retinal dystrophies, ischemic insult of retinal neurons and macular edema.

Described herein are methods for the prevention and/or treatment of retinal diseases selected from age-related macular degeneration, retinal vasculitis and retinal infective processes, commotio retinae, diabetic retinopathy, hereditary retinal dystrophies, ischemic insult of retinal neurons and macular edema; wherein said prevention or treatment of retinal diseases is ensured by infusion into the intrathecal space, subarachnoid space or cerebral ventricles of a composition comprising CSF or artificial CSF (aCSF) comprising sodium ions at a concentration of 140-190 mM, potassium ions at a concentration of 2.5-4.5 mM, calcium ions at a concentration of 1-1.5 mM, magnesium ions at a concentration of 0.5-1.5 mM, phosphor ions at a concentration of 0.5-1.5 mM, chloride ions a concentration of 100-200 mM. In particular, the methods comprise increasing glymphatic system clearance in the optic nerve and/or retina by the administration of CSF or aCSF for increasing or promoting glymphatic system clearance.

In particular embodiments, the methods involve the administration of said composition comprising CSF or CSF-like composition to the intrathecal space or subarachnoid space or the cerebral ventricles of said patient to facilitate glymphatic clearance in the optic nerve and/or retina. Administration of said composition comprising CSF or CSF-like composition can be ensured by an implantable pump.

The principle of the methods described herein is based on the observation that the human optic nerve comprises a paravascular circulation. Provided herein is the administration of CSF or artificial CSF as defined above, by infusion into the intrathecal or subarachnoid space or cerebral ventricles for increasing or promoting glymphatic system clearance for the treatment and/or prevention of the retinal diseases listed above in a patient. This provides a protective effect for the optic nerve and/or retina by increasing glymphatic system clearance in the optic nerve and/or retina.

Described but not claimed herein is a therapeutic agent for increasing or promoting glymphatic system clearance in the optic nerve and/or retina for use in the prevention and/or treatment of retinal diseases in a patient. In addition, it has been observed that patients suffering from or likely to develop glaucoma can also benefit from this treatment method. In particular aspects, said therapeutic agent is selected from a diuretic, an adrenergic receptor antagonist, a Stat-3 inhibitor, a bone morphogenetic protein (BMP) signaling axis molecule, a vasopressin (AVP) antagonist, an antagonist of atrial natriuretic peptide (ANP), an Angiotensin II antagonist, an AT2R receptor antagonist, an AT1 receptor antagonist, an agent for use in the treatment of insomnia or as an aid for sleep and an agent that prevents AQP4 depolarization or loss of AQP4 polarization. In particular aspects, said therapeutic agent is an antagonist of AVP such as tolvaptan, conivaptan or VPA-985. In particular aspects, said therapeutic agent is an antagonist of atrial natriuretic peptide (ANP) such as anantin. In particular aspects, said therapeutic agent is and angiotensin II antagonist such as losartan. In particular aspects, said therapeutic agent is an antagonist of AT2R receptors such as PD 123319. In particular aspects, said therapeutic agent is an antagonist of AT1 receptors such as valsartan.

Said therapeutic agent for use in the methods described but not claimed herein can be an agent that prevents AQP4 depolarization or loss of AQP4 polarization such as JNJ-17299425 or JNJ-17306861. In particular aspects, said therapeutic agent is agent for use in the treatment of insomnia or as aid for sleep, such as antihistamines (e.g., over-the-counter), non-prescription sleep aids, Benzodiazepines, non-Benzodiazepines, melatonin receptor stimulators or barbiturates. In particular aspects, said therapeutic agent is selected from ALLEGRA^{®} (Fexofenadine), BENADRYL^{®} (Diphenhydramine), CLARITIN^{®} or TAVIST^{®} (loratadine), CHLOR-TRIMETON^{®} (chlorpheniramine maleate), DIMETANE^{®} (Brompheniramine, Phenylpropanolamine) and ZYRTEC^{®} (Cetirizine). In particular aspects, said therapeutic agent is selected from Unisom Nighttime Sleep-Aid, Dormin, Nytol, Simply Sleep, Sominex, Extra Strength Tylenol PM, Diphenhydramine hydrochloride and Excedrin P.M.., benzodiazepines, non-benzodiazepines, imidazopyridines or barbiturates.

The above and other characteristics, features and advantages of the present invention will become apparent from the following detailed description, which illustrates, by way of example, the principles of the invention.

### BRIEF DESCRIPTION OF THE DRAWINGS

The following description of the figures of specific embodiments of the invention is merely exemplary in nature and is not intended to limit the present teachings, their application or uses. Throughout the drawings, corresponding reference numerals indicate like or corresponding parts and features.
**Figure 1****.** (HE, 50x). Histopathology demonstrating dura mater with lymphatics and subarachnoid space both filled with indian ink (HE, 50x).
**Figure 2****.** Cross-section through the optic nerve (HE, 100x): Distribution of ink in the surroundings of the blood vessels in the optic nerve showing the complex slit-like space.
**Figure 3.** 3a (HE, 200x), Fig. 3b (HE, 400x) Cross-section through the optic nerve (A: HE, 200x; B: HE, 400x): Distribution of ink in the surroundings of the central retinal vessels in the optic nerve showing the complex slit-like space, in higher magnification demonstrating the dye around the central retinal artery between collagen fiber bundles.
**Figure 4****.** Cross-section through the optic nerve (Holmes-Luxol, 400x): Distribution of ink in the surroundings of the central retinal artery showing the complex slit-like space.

### DETAILED DESCRIPTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited components, elements or method steps also include embodiments which "consist of" said recited components, elements or method steps.

Furthermore, the terms "first", "second", "third" and the "like" in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment. Furthermore, the particular features, structures or characteristics may be combined in any suitable manner, as would be apparent to a person skilled in the art from this disclosure, in one or more embodiments.

For example, in the appended claims, any of the features of the claimed embodiments can be used in any combination.

The values as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that each value as used herein is itself also specifically, and preferably, disclosed.

The recitation of numerical ranges by endpoints includes all numbers and fractions subsumed within the respective ranges, as well as the recited endpoints.

The glymphatic system was first described by Iliff et al. in 2012 (Iliff et al., Sci Transl Med. 2012;4:147ra111). The authors defined for the first time a brain-wide network of paravascular channels, which they termed the 'glymphatic pathway', along which CSF moves into and through the brain parenchyma, facilitating the exchange of CSF and ISF and the clearance of interstitial solutes from the brain. Accordingly, increasing or promoting the glymphatic clearance system facilitates clearance of waste products from the brain, such as amyloid-β.

The Inventors discovered a fluid circulation in the human optic nerve, which occurs via such paravascular pathways. In a post-mortem study, the Inventors discovered paravascular spaces around the central retinal artery and vein in the human optic nerve. Furthermore, it appears that increased glymphatic clearance, whereby the CSF turnover and clearance of waste products from the optic nerve is promoted, can prevent and/or treat glaucoma. Accordingly, therapeutic agents which are able to increase or promote the glympathic system clearance can be used to treat or prevent glaucoma. Moreover, there seems to be a paravascular communication between the surroundings of the retinal vascular system and the central retinal artery and vein in the optic nerve. Accordingly, therapeutic agents which are able to increase or promote the glympathic system clearance could be used to treat or prevent retinal diseases.

The term 'glymphatic system' as used herein refers to a waste clearance system that utilizes a unique system of paravascular tunnels to promote efficient elimination of interstitial solutes, including amyloid-beta.

The term "retinal disease" as used herein refers to a disease primarily affecting the retina. Examples include age-related macular degeneration, retinal vasculitis and retinal infective processes, commotio retinae (as a result of blunt ocular trauma), diabetic retinopathy, hereditary retinal dystrophies, ischemic insult of retinal neurons and macular edema. In particular embodiments, the retinal disease is a non-proliferative retinal disease.

The term "glaucoma" as used herein according to its well-established meaning. Glaucoma is a disease that damages the eye's optic nerve. It is often a result of fluid building up in the front part of the eye. That extra fluid increases the pressure in the eye, damaging the optic nerve. It is noted that Glaucoma is not considered a retinal disease. Indeed, According to the International Society of Geographical and Epidemiological Ophthalmology (ISGEO) classification, primary open-angle glaucoma is defined as glaucomatous optic neuropathy in the presence of an open-angle and no other ocular abnormality to account for a secondary mechanism. The optic nerve, beginning at the optic nerve head and ending at the optic chiasm, consists of the efferent axons of the retinal ganglion cells. Raised intraocular pressure (IOP) is a major risk factor in glaucoma, and evidence from experimental studies in primates and rodents strongly indicates that the site of lOP-induced axonal damage in glaucoma is at the optic nerve head. The optic nerve head contains a central cup. As glaucoma progresses, the cup of the optic nerve deepens and becomes undermined. The lamina cribrosa, which is located at the bottom of the cup of the optic nerve, is composed of a series of sieve-like collagenous plates in the optic nerve head. Postmortem examination of glaucomatous eyes shows that thinning of the lamina cribrosa and deformity of the laminar pores compress the retinal ganglion cell axons, which form the optic nerve (Quigley HA, et al. Arch Ophthalmol 1981;99:137-43). Therefore, glaucoma has traditionally been classified as an optic neuropathy and not as a retinal disease, although the direct damage to the optic nerve axons causes changes in retinal ganglion cells, eventually inducing cell death in the course of the disease.

The term 'therapeutic agent' as used herein refers to a substance capable of producing a beneficial and desirable effect when consumed, more particularly, increasing or promoting the glymphatic system clearance, thereby preventing and/or treating glaucoma and/or retinal diseases.

CSF flows from the brain ventricles into interconnecting chambers, namely, the cisterns and the subarachnoid spaces, including the SAS of the optic nerves. The optic nerve, a white matter tract of the central nervous system, is ensheathed in all three meningeal layers and surrounded by CSF in the SAS with a pressure equivalent to intracranial pressure (ICP). Thus, in addition to intraocular pressure (IOP), the optic nerve is exposed to the ICP. The lamina cribrosa, the perforated region of the sclera through which the nerve fibers of the optic nerve pass as they exit the eye, separates these two pressurized regions. The difference between the posteriorly directed IOP and anteriorly directed ICP across the lamina cribrosa is known as the trans-lamina cribrosa pressure difference (TLCPD).The term "intracranial pressure" or "ICP" as used herein thus refers to the pressure of CSF within the skull and thus in the brain tissue and CSF and is also referred to as "CSF pressure". The CSF pressure as assessed by lumbar puncture correlates with ICP, and thus the terms CSF pressure and ICP are used interchangeably. The ICP is built up by the equilibrium between the production and outflow of CSF. If the intracranial compliance is assumed to be constant, the steady-state ICP can be described by a simplified equation: ICP = I_{f} x Rₒᵤₜ + Pₛₛ, where I_{f} is CSF formation rate, Rₒᵤₜ is outflow resistance, and Pₛₛ is sagittal sinus pressure. ICP is measured in millimetres of mercury (mmHg). At rest it is normally between 5-15 mmg Hg for an adult when measured by lumbar puncture in the lateral decubitus position. Accordingly, the values of ICP (or CSF pressure) referred to herein refer to values when measured in the lateral decubitus position.

The term "intraocular pressure" or "IOP" as used herein refers to the fluid pressure within the eye. It is measured in millimetres of mercury (mmHg). Normally the lOP ranges from 11 to 21 mmHg with a mean of 16 mmHg.

The "trans-lamina cribrosa pressure difference" or "TLCPD" is the difference between the posteriorly directed IOP and the anteriorly directed ICP across the lamina cribrosa.

The pressure drop that occurs across the lamina cribrosa (IOP-ICP) increases with elevation of IOP or reduction of ICP. Indeed, from a mechanical perspective, a similar posteriorly directed force is caused by either a lower pressure on the CSF side of the lamina or a higher pressure on the intraocular side.

A "CSF-like solution" as used herein refers to a solution that consists essentially of CSF or artificial CSF.

The term "artificial CSF" (aCSF) as used herein refers to a solution that closely matches the electrolyte concentrations of cerebrospinal fluid. Typically, the artificial CSF comprises sodium ions at a concentration of 140-190 mM, potassium ions at a concentration of 2.5-4.5 mM, calcium ions at a concentration of 1-1.5 mM, magnesium ions at a concentration of 0.5-1.5 mM, phosphor ions at a concentration of 0.5-1.5 mM, chloride ions a concentration of 100-200 mM. In one example, the artificial CSF comprises sodium ions at a concentration of 150 mM, potassium ions at a concentration of 3 mM, calcium ions at a concentration of 1.4 mM, magnesium ions at a concentration of 0.8 mM, phosphor ions at a concentration of 1 mM, chloride ions a concentration of 155 mM. aCSFs have been described in the art and include, but are not limited to Elliot's solutions A and B and ARTCEREB ^{™}.

Typically where reference is made to the administration of CSF, it is intended to refer to a CSF-like solution or to CSF which is (at least partially) of foreign origin (i.e. not from the patient).

In particular embodiments, the CSF may further comprise one or more therapeutic agents, for example agents for reducing the IOP and/or increasing the ICP and/or stimulating the CSF production and/or increasing CSF turnover and/or increasing glymphatic clearance. For example specific peptides such as angiotensin have been shown to facilitate the rise in CSF pressure upon CSF infusion.

The term "intrathecal space" also referred to as SAS is the fluid-filled area located between the innermost layer of covering (the pia mater) of the spinal cord and the middle layer of covering (the arachnoid mater).

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Provided herein are compositions comprising CSF or artificial CSF comprising sodium ions at a concentration of 140-190 mM, potassium ions at a concentration of 2.5-4.5 mM, calcium ions at a concentration of 1-1.5 mM, magnesium ions at a concentration of 0.5-1.5 mM, phosphor ions at a concentration of 0.5-1.5 mM, chloride ions a concentration of 100-200 mM for use in treating and/or preventing a retinal disease selected from age-related macular degeneration, retinal vasculitis and retinal infective processes, commotio retinae, diabetic retinopathy, hereditary retinal dystrophies, ischemic insult of retinal neurons and macular edemaby by infusion into the intrathecal space, subarachnoid space or cerebral ventricles.

More particularly, the compositions for use as provided herein ensure an increase in glymphatic system clearance in the optic nerve and/or retina. An optimal CSF turnover and an efficient CSF-ISF exchange in the optic nerve, allowing clearance of interstitial solutes and metabolic wastes, such as amyloid-β, may be vital for the integrity of the optic nerve. The turnover of CSF and glymphatic transport decrease substantially with aging and thus the degree of increase in CSF turnover and glymphatic transport will need to take into consideration the age of the patient. In a young adult, it is envisaged that the turnover is ideally about 4.0 volumes/day. The compositions for use as described herein can be administered to a patient for increasing or promoting glymphatic system clearance.

By delivering said CSF or aCSF for increasing or promoting glymphatic system clearance, the clearance in the optic nerve and/or retina are increased (thereby enhancing removal of potentially neurotoxic waste products that accumulate in the optic nerve and/or retina), thus ensuring the treatment or prevention of the retinal diseases listed above.

In particular embodiments, the composition comprising CSF or aCSF for use as claimed herein is administered after the disease has been diagnosed to ensure a therapeutic effect.

In further embodiments, the composition comprising CSF or aCSF for use as claimed herein is administered to a subject at risk of developing a retinal disease listed above, whereby a prophylactic effect i.e; an effect on the development of the disease is envisaged.

In particular embodiments, the compositions are provided for use in a method for prevention of a retinal disease listed above and the patient is a patient at risk of developing retinal diseases.

In further embodiments, the compositions are envisaged for use in a method of treatment of a retinal disease listed above and the patient is diagnosed as suffering from said retinal disease.

Glaucoma can be diagnosed or the risk of developing glaucoma can be determined using different assay systems. An exemplary method is a diagnosis by an eye pressure test using a tonometer to measure the pressure inside the eye. In this way the intraocular pressure can be measured, so as to determine ocular hypertension, which is a risk factor for chronic open-angle glaucoma. Further, the measurement of corneal thickness can be performed as an indication of intra-ocular pressure. Gonioscopy is the examination of the front outer edge of the eye, between the cornea and the iris, where the fluid typically drains out of the eye, to determine whether this angle is open or closed. Finally a visual field test can be used to determine whether or not there is visual impairment, more particularly, missing areas of vision. Typically, dots appear in the peripheral vision where glaucoma begins. Where glaucoma has affected vision, these spots are no longer visible. The optic nerve head can be examined using a direct ophthalmoscope, an indirect ophthalmoscope, or using a posterior pole lens with a slit lamp. Dilating the pupil facilitates and improves the accuracy of the examination with all instruments. More sophisticated devices such as scanning laser polarimetry, confocal scanning laser ophthalmoscopy, and ocular coherence tomography can also be used to complement the clinical examination of the optic disc and provide quantitative measurements.

Described but not claimed herein are therapeutic agent for increasing or promoting glymphatic system clearance in the optic nerve and/or retina for use in the prevention and/or treatment of glaucoma, ocular hypertension and/or retinal diseases in a patient. In particular aspects, the increased glymphatic system clearance in the retina is achieved by pumping fluid through the central nervous system interstitium. In particular aspects, the therapeutic agents described but not claimed herein are for use in the prevention of glaucoma in a patient at risk of developing glaucoma. Patients at risk of developing glaucoma include but are not limited to patients suffering from diabetes, high blood pressure, use of corticosteroids, having undergone eye surgery or injury, having high myopia and/or having a direct relative suffering from glaucoma. In particular aspects, methods are provided which involve treating a patient at risk or developing glaucoma. In particular aspects, the glaucoma is normal tension glaucoma. In particular aspects, the therapeutic agents described but not claimed herein are for use in the prevention of retinal diseases in a patient at risk of developing retinal diseases. Patients at risk of developing retinal disease include but are not limited to patients suffering from a blunt trauma or infective, auto-immune, inflammatory or neoplastic disorders.

The therapeutic agents described but not claimed herein can be any agent that increases or promotes glymphatic system clearance, preferably without causing any severe side effects. The therapeutic agent can be of natural or synthetic origin, include active agents such as drugs or hormones, or active agents coupled to an appropriate carrier. The therapeutic agent is typically administered systemically. This can be achieved via enteral (e.g. oral intake) or parenteral administration (e.g. injection, infusion or implantation). In particular embodiments, the therapeutic agent is administered via intrathecal administration and/or via intravitreal injection and/or via topical application as eye drops. The therapeutic agent for use described but not claimed herein can be for use at an effective dosage. As used herein, an "effective dosage" or "effective amount" of the therapeutic agent is the dosage of the therapeutic agent sufficient to effect beneficial or desired results. Herein, beneficial or desired results include results such as suppressing or reducing the onset and/or development of glaucoma and/or elevated intraocular pressure or decreasing one or more symptoms resulting from glaucoma and/or reducing the onset and/or development of retinal diseases. An effective dosage can be administered in one or more administrations. In particular aspects, the timing of the administration of the therapeutic agent is tuned in such a way that the therapeutic agent reaches a consistent level in the bloodstream. In particular aspects, the effective dosage of the therapeutic agent ranges from about 0.1 mg to 1000 mg, from about 0.1 mg to 500 mg, from about 0.1 mg to 250 mg, from about 0.1 mg to 100 mg, from about 0.1 mg to 50 mg, from about 0.1 mg to 25 mg, from about 0.1 mg to 10 mg per day, from about 0.1 to 5 mg per day, from about 0.1 to 2.5 mg per day or from about 0.1 to 1 mg per day. In particular aspects, the effective dosage of the therapeutic agent ranges from about 1 mg to 1000 mg, from about 1 mg to 500 mg, from about 1 mg to 250 mg, from about 1 mg to 100 mg, from about 1 mg to 50 mg, from about 1 mg to 25 mg, from about 1 mg to 10 mg per day. In particular aspects, the therapeutic agent is administered at a dosage of between 5nM and 100µM, more particularly at a dosage between 10 nM and 10µM, such as 1 µM.

Described but not claimed herein are therapeutic agents as described in patent application US 2016/0000945 A1. The therapeutic agent described but not claimed herein can be a diuretic, an adrenergic receptor antagonist, a Stat-3 inhibitor or molecules known in the art to be bone morphogenetic protein (BMP) signaling axis molecules, an antagonist of AVP (vasopressin) such as tolvaptan, conivaptan, or VPA-985, an antagonist of atrial natriuretic peptide (ANP) such as anantin, an antagonist of Angiotensin II such as losartan, an antagonist of AT2R receptors such as PD 123319, an antagonist of AT1 receptors such as valsartan, an agent that prevents AQP4 depolarization or loss of AQP4 polarization such as JNJ-17299425 or JNJ-17306861, or an agent for use in the treatment of insomnia or as aid for sleep, such as antihistamines (e.g., over-the-counter), non-prescription sleep aids, Benzodiazepines, non-Benzodiazepines, melatonin receptor stimulators or barbiturates.

Non-limiting examples of antihistamines are ALLEGRA^{®} (Fexofenadine), BENADRYL^{®} (Diphenhydramine), CLARITIN^{®} or TAVIST^{®} (loratadine), CHLOR-TRIMETON^{®} (chlorpheniramine maleate), DIMETANE^{®} (Brompheniramine, Phenylpropanolamine) and ZYRTEC^{®} (Cetirizine).

Non-limiting examples of non-prescription sleep aids are Unisom Nighttime Sleep-Aid, Dormin, Nytol, Simply Sleep, Sominex, Extra Strength Tylenol PM, Diphenhydramine hydrochloride and Excedrin P.M.., benzodiazepines, non-benzodiazepines, imidazopyridines or barbiturates.

Non-limiting examples of Benzodiazepines are PROSUM^{®} (estazolam), DALMANE^{®} (flurazepam), DORAL^{®} (quazepam), RESTORIL^{®} (temazepam), HALCION^{®} (triazolam) and VALIUM^{®} (diazepam).

Non-limiting examples of non-Benzodiazepines are Imidazopyridines (e.g. AMBIEN^{®}, AMBIEN^{®} CR, INTERMEZZO^{®} (zolpidem)) and SONATA^{®} (pyrazolopyrimidine). Non-limiting examples of melatonin receptor stimulators are ROZEREM^{®} (ramelteon), NOTEC^{®} (chloral hydrate), PRECEDEX^{®} (dexmedetomidine hydrochloride) and LUNESTA^{®} (eszopiclone).

Non-limiting examples of barbiturates are NEMBUTAL^{®} (phenobarbital), MEBARAL^{®} (mephobarbital), Amytal Sodium (amobarbital sodium), BUTISOL^{®} (butabarbital sodium) and SECONAL^{®} Sodium Pulvules (secobarbital sodium).

Described but not claimed herein are said therapeutic agent for increasing or promoting glymphatic system clearance in the optic nerve and/or retina as described herein is administered in combination with a pharmaceutical carrier or adjuvant which facilitates administration of said therapeutic agent to the intrathecal space, subarachnoid space or the cerebral ventricles of said patient.

The present inventor has previously found that reduced ICP contributes to glaucoma and retinal diseases via a mismatch in pressures across the lamina cribrosa (TLCPD), such that lowering the TLCPD by manipulation of ICP by infusion of CSF can be used to prevent and/or treat glaucoma or retinal diseases. Moreover, said infusion of CSF allows an enhancement of the rate of CSF turnover and increases glymphatic clearance in the optic nerve and/or retina which is believed to provide an additional or alternative beneficial effect for the prevention and treatment of glaucoma and/or retinal diseases. More specifically, administration of CSF or a CSF-like solution (such as artificial CSF) directly or indirectly to the cerebral ventricles and/or to the intrathecal space around the spinal cord leads to an increase of the ICP (or decrease of the TLCPD) and/or increase of the rate of CSF turnover and clearance in the subarachnoid space of the optic nerve and/or an increase in glymphatic clearance in the optic nerve and/or retina (thereby enhancing removal of potentially neurotoxic waste products that accumulate in the optic nerve and/or retina), thus ensuring the treatment of glaucoma and/or retinal diseases. Indeed, in a non-claimed aspect, glaucoma can be prevented or treated from the intracranial compartment side of the lamina instead of, or in addition to, lowering IOP.

Accordingly, provided herein are compositions for use in methods of prevention and/or treatment of retinal diseases selected from age-related macular degeneration, retinal vasculitis and retinal infective processes, commotio retinae, diabetic retinopathy, hereditary retinal dystrophies, ischemic insult of retinal neurons and macular edema in a patient in need thereof comprising CSF or artificial CSF comprising sodium ions at a concentration of 140-190 mM, potassium ions at a concentration of 2.5-4.5 mM, calcium ions at a concentration of 1-1.5 mM, magnesium ions at a concentration of 0.5-1.5 mM, phosphor ions at a concentration of 0.5-1.5 mM, chloride ions a concentration of 100-200 mM.

Described but not claimed herein are the compositions as described above for use in a method wherein said patient which is developing or suffering from glaucoma and/or retinal diseases is administered simultaneously or sequentially, a composition comprising CSF or a CSF-like composition and said therapeutic agent for increasing or promoting glymphatic system clearance in the optic nerve and/or retina as described above.Said composition comprising CSF or aCSF as defined above can be administered after the administration of said therapeutic agent. Said composition comprising CSF or aCSF can be administered before the administration of said therapeutic agent.

Said composition comprising CSF aCSF as defined above is administered to the intrathecal space, subarachnoid space or the cerebral ventricles of said patient. More particularly, the artificial CSF can be administered to the intrathecal space surrounding the spinal cord. Indeed, the administration of CSF or aCSF as defined above can be done locally, in the vicinity of or in the subarachnoid space of the optic nerve, but the same effect can be achieved less invasively by infusion more remotely, i.e. intrathecally anywhere along the spinal cord, including the cervical region, the thoracic region, the lumbar region etc.

Described but not claimed herein are said compositions as defined above for use in methods wherein said therapeutic agent for increasing or promoting glymphatic system clearance in the optic nerve and/or retina is delivered systemically and said composition comprising CSF or CSF-like composition is administered to the intrathecal space, subarachnoid space or the cerebral ventricles of said patient.

Described but not claimed herein are said compositions as defined above for use in methods wherein both said therapeutic agent for increasing or promoting glymphatic system clearance in the optic nerve and/or retina and said composition comprising CSF or CSF-like composition are administered to the intrathecal space, subarachnoid space or the cerebral ventricles of said patient.

In particular embodiments of the compositions for use as claimed herein, the administration with CSF or a CSF-like solution ensures an increase in CSF turnover and/or an increase in glymphatic clearance in the optic nerve and/or retina. Indeed it is considered that the increase in CSF turnover ensures the therapeutic effect. According to a particular embodiment, this is ensured by ensuring maximal CSF infusion. While not critical to the present invention, in particular embodiments, the administration of CSF or a CSF-like solution by infusion reduces the TLCPD, preferably to less than 4mm Hg, such as to 1 or 2 mmHg. Indeed, maximal CSF infusion will be reached when the TLCPD reaches a minimal value without conversion of the TCLPD (ICP becomes higher than the IOP), while at the same time ICP should not become excessively high to avoid brain damage. Accordingly, in particular embodiments, the compositions for use as claimed herein are for use in methods which comprise establishing the maximal CSF infusion rate without TCLPD conversion or excessive increase of ICP. This is explained more in detail below. Increasing intracranial pressure (ICP) will facilitate ocular paravascular glymphatic inflow. Normally, intraocular pressure (IOP) exceeds intracranial pressure (ICP), and on average there is a small force (mean 4 mmHg) directed posteriorly across the lamina cribrosa. This trans-lamina cribrosa pressure difference (TLCPD) (IOP-ICP) may ensure effective paravascular outflow from the eye. However, in case of reversal of the TLCPD (ICP>IOP), ocular paravascular outflow may be completely impeded. This can result in glymphatic stasis predominantly within the prelaminar region of the optic nerve head, and this could contribute to the optic disc edema. For these reasons there should not be a reversion of the TLCPD as this would block the glymphatic outflow from the eye. Accordingly a minimal TLCPD of about 1 to 2 mm Hg is ideally maintained. At the same time, cerebral complications should be avoided. Thus, an excessive increase of the intracranial pressure (ICP) will need to be avoided. Accordingly, in particular embodiments, an ICP pressure of between 20 cm H₂O (14,7 mm Hg) and 25 cm H₂O (18,3 mm Hg) is considered borderline while an ICP value of over 25 cm H₂O (18,3 mm Hg) should be avoided. Accordingly when using a pump it can be envisaged to ensure that the CSF pressure does not exceed a given value such as 18 mm Hg, or even preferably does not exceed 15 mm Hg.

In particular embodiments, compositions for use as claimed herein wherein the administration with CSF or aCSF are envisage for use in methods wherien increasing or promoting glymphatic system clearance in the optic nerve and/or retina as described herein ensured by way of an implantable apparatus configured for infusing fluid, more particularly into the intrathecal or subarachnoid space of said patient.

Such an apparatus can be an infusion pump for infusing CSF or a CSF-like solution into a body cavity, more particularly into the intrathecal or subarachnoid space. In non-claimed aspects, the apparatus can be used for the treatment and/or prevention of glaucoma, more particularly open-angle glaucoma, and/or for the treatment and/or prevention of retinal diseases, such as, but not limited to, age-related macular degeneration, retinal vasculitis and commotio retinae. Intrathecal infusion pumps are currently widely used for management of chronic pain (morphine pump) and spasticity (baclofen pump). The apparatus for infusing fluid into the intrathecal or subarachnoid space of a patient can comprise an implantable pump, a reservoir for containing artificial cerebrospinal fluid, an infusion catheter having an inlet end coupled to the reservoir, and an outlet end coupled to the implantable pump and an inflow catheter. The inflow catheter can have an outlet end configured to be disposed in fluid communication with said intrathecal or subarachnoid space, and an inlet end coupled to the implantable pump; typically the implantable pump is configured to selectively move artificial cerebrospinal fluid from the reservoir through the infusion catheter and the inflow catheter to the intrathecal or subarachnoid space at a rate and volume sufficient to increase the intracranial pressure and/or the cerebrospinal fluid turnover and/or glymphatic clearance in the optic nerve and/or retina in a patient. More particularly the rate and volume of CSF infusion are adjusted so as to reduce TLCPD to a value of about 4mm Hg or less, or even to 2 mm Hg or 1 mm Hg (dependent on the patient). In particular cases, the rate and volume of artificial CSF infusion ensures that an ICP of between 11 and 16 mm Hg is maintained, more particularly that it does not exceed 15 mm Hg. In particular embodiments, the pump is a pump as described in WO2016/059162.

The infusion rate ensured by the pump will be determined based on different factors, including the CSF absorption rate of the patient. The infusion rate can be adjusted to ensure an increased turnover of CSF in the patient. The pump can configured to ensure a CSF infusion rate in the range of 0.05-0.1 ml/min, 0.1-0.2 ml/min, 0.2-0.42 ml/min, 0.42-0.7ml/min or even up to 0.7-1.04 ml/min (1.5 L/day). The infusion rate is able to ensure a turnover of about 4.0 volumes/day.

Furthermore, the apparatus can comprise a microcontroller that controls operation of the implantable pump. Said microcontroller can regulate the flow of the CSF or CSF-like solution through the inflow catheter.

Moreover, the apparatus can comprise a flow sensor disposed in communication with the inflow catheter to monitor the volume and flow rate of artificial CSF pumped into the intrathecal space or cerebral ventricles, wherein the microcontroller is configured to activate the implantable pump responsive to an output of the pressure sensor. Additionally, the apparatus can be combined with an implantable pressure sensor to monitor intracranial or CSF pressure in the patient. The sensor does not need to be physically linked to the rest of the apparatus but can be implanted intrathecally or in the cerebral ventricles. The microcontroller of the apparatus can be configured to activate or deactivate the implantable pump responsive to an output of the pressure sensor. For example, the microcontroller can be configured to a constant intracranial pressure. Moreover, the apparatus can comprise a feedback mechanism based on the output of the pressure sensor, which ensures that the intracranial pressure does not exceed a certain value, which is preferably 15 mm Hg, but can be up to 18 mm Hg.

Although the implantation of a CSF pump is a relatively invasive intervention, it provides a worthwhile alternative for or addition to existing therapies, especially for patients for whom non-invasive treatment options are ineffective.

For example, the apparatus comprises an implantable pump, a reservoir for containing artificial cerebrospinal fluid, an infusion catheter having an inlet end coupled to the reservoir, and an outlet end coupled to the implantable pump; and an inflow catheter having an outlet end configured to be disposed in fluid communication with said intrathecal or subarachnoid space or the cerebral ventricles, and an inlet and is used in the following manner:
- providing cerebrospinal fluid or a CSF-like solution in said reservoir;
- coupling the inflow catheter to a region of a body cavity, more particularly an intrathecal space or the cerebral ventricles; and
- activating the implantable pump to pump the cerebrospinal fluid or CSF-like fluid from the reservoir through the infusion catheter and the inflow catheter to the body cavity (e.g. the subarachnoid space, one of the lateral ventricles, or the central canal of the spinal cord) at a rate and volume sufficient to increase the ICP and/or to increase the CSF turnover and/or to increase glymphatic clearance in the optic nerve and/or retina.

The pump can be surgically placed under the skin of the abdomen, and delivers small, CSF or CSF-like fluid through a catheter directly into the CSF locally present. The present inventor has found previously that such pumps may be provided for infusing artificial CSF,

in order to increase ICP and/or CSF turnover with the aim of treating glaucoma. Optionally, the pump as described herein can be used to deliver both the CSF (or CSF-like fluid) and the therapeutic agent for increasing or promoting glymphatic system clearance in the optic nerve and/or retina as described herein.

In a non-claimed aspect, the methods described herein are particularly suitable for the prevention and/or treatment of glaucoma and/or retinal diseases, such as age-related macular degeneration, retinal vasculitis and commotio retinae. Prevention of glaucoma can be envisaged in patients susceptible to glaucoma such as patients having reduced intracranial pressure and/or increased TLCPD. Examples of risk factors associated with glaucoma include but are not limited to elevated IOP, low ICP, age, gender, high myopica etc. Long term use of topical and systemic steroids produces secondary open-angle glaucoma by causing an increase in IOP.

In particular non-claimed aspects, the application envisages the compositions for use in methods which further involve determining one or more of the IOP, TLCPD and/or ICP in a patient prior to the administration according to the methods described herein. This step can be ensured in order to determine the suitability of the methods described herein for the prevention and/or treatment of glaucoma. Additionally or alternatively it can be used to determine the optimal infusion rate of CSF.

Methods applied for non-invasive estimation of ICP are known in the art and include transcranial Doppler ultrasonography, tympanic membrane displacement, ophthalmodynamometry, measurement of the orbital CSF space around the optic nerve, two-depth transcranial Doppler technology and others. Two-Depth Transorbital Doppler (TDTD) measurement of intracranial pressure quantitative absolute (ICP) value relies on the same fundamental principle as used to measure blood pressure with a sphygmomanometer. The TDTD method uses Doppler ultrasound to translate pressure balance principle of blood pressure measurement with a sphygmomanometer to the measurement of ICP. The ophthalmic artery (OA), which is a vessel with intracranial and extracranial segments, is used as pressure sensor and as a natural pair of scales for absolute ICP value in mmHg or mmH₂O measurement. Blood flow in the intracranial OA segment is affected by intracranial pressure, while flow in the extracranial (intraotbital) OA segment is influenced by the externally applied pressure (Pe) to the eyeball and orbital tissues.

The present invention is further illustrated in the following non-limiting examples.

### EXAMPLES

### Example 1: Evidence for paravascular spaces in the human optic nerve

### Material and methods

This post-mortem study was carried out in seven subjects without ocular disease. The samples were obtained no later than 6 hours post-mortem, following qualified consent for autopsy.

### Preparation and Labelling of Samples

All optic nerves including the globe were removed after removal of the orbital roof. The optic nerves were ligated with a 6.0 silk suture proximal to the optic chiasm. The fixative (neutral buffered 4% formalin) was then slowly injected into the SAS with a 19-gauge needle. Care was taken to avoid high-injection pressure in order not to create artefacts. In two cases, indian ink dissolved in 8% formalin (vol:vol = 1:1) was injected slowly under low pressure into the SAS of the optic nerve. The samples were fixed in 4% formalin by immersion for 5 days before further work-up.

### Light Microscopy

From each optic nerve, a piece including the midorbital portion and bulbar segment (adjacent to the globe) was processed for paraffin blocks and cut in sections 5-8 µm thick. The stains used included haematoxylin and eosin, van Gieson elastin, and Masson trichrome.

### Results

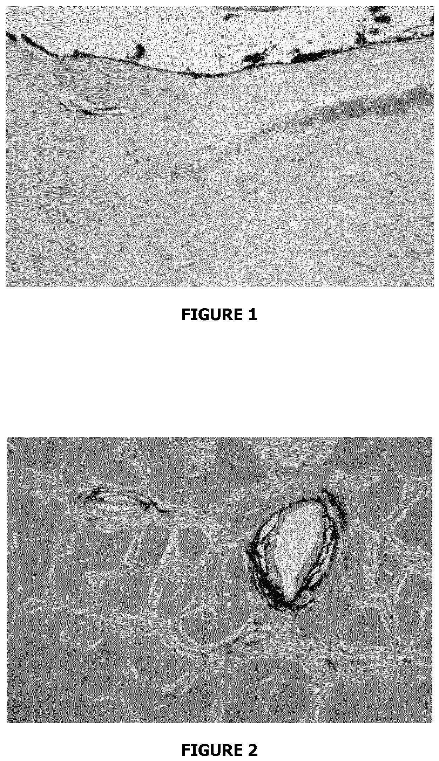

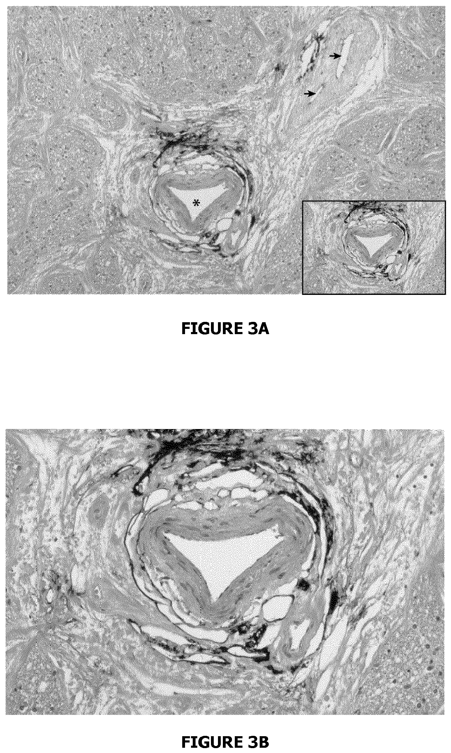

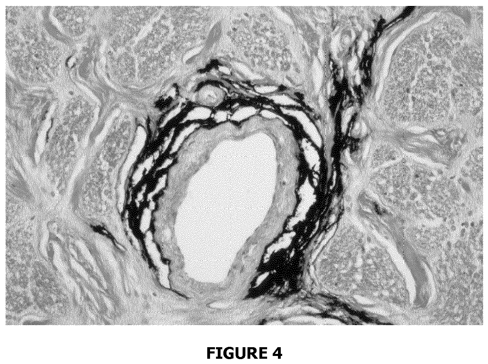

Indian ink was injected into the subarachnoid space (SAS) of the human optic nerve to study possible paravascular fluid circulation. Using light microscopy, the Inventors found that the SAS was filled with indian ink. From here the dye drained into the lymphatics of the dura mater of the optic nerve (Figure 1). Moreover, the indian ink travelled from the SAS to the paravascular space around the central retinal vessels. A high amount of deposits was detectable in the surroundings of the blood vessels in the optic nerve (Figure 2, Figure 3a, Figure 3b and Figure 4), whereas the lumens of these vessels were unlabeled. The present inventors provide evidence for a new clearance pathway in the human optic nerve. In light of the key role that the glymphatic pathway may play in the clearance of interstitial solutes from the brain, the observation of such an anatomically distinct clearing system in the optic nerve is of great importance for understanding how solutes are cleared from the ISF in the optic nerve, and provides new insights into the pathogenesis of conditions such as glaucoma. Indeed, one might expect that a dysfunctional glymphatic system could ultimately result in reduced neurotoxin clearance in the optic nerve and lead to glaucomatous neurodegeneration. Moreover, there seems to be a paravascular communication between the surroundings of the retinal vascular system and the central retinal artery and vein in the optic nerve. Accordingly, a dysfunctional glymphatic system could also contribute to retinal diseases.

### Example 2: Therapeutic agents for increasing or promoting glymphatic system clearance can treat glaucoma in rats (Reference example)

A glaucoma rat model is used to study the effect of one or more therapeutic agents for increasing or promoting glymphatic system clearance on the prevention and/or treatment of glaucoma.

The rats are either treated with one or more therapeutic agents for increasing or promoting glymphatic system or vehicle. Systemic administration is performed, optionally repetitively, before the onset of glaucoma to evaluate a potential preventive effect or upon occurrence of clinical symptoms of glaucoma (active phase) to evaluate a potential curative effect. An antagonist of Angiotensin II such as losartan, an antagonist of vasopressin or an antagonist of atrial natriuretic peptide (ANP) is used at a concentration of 1 µM. Hereafter, rats are sacrificed and eyes and optic nerves are dissected out and fixed for staining and analysis of optic nerve and retinal ganglion cell degeneration. Treatment is successful if retinal ganglion cell axon death is significantly less in the treatment group than in control glaucoma eyes.

### Example 3: Intracranial CSF infusion in an animal model of age-related macular degeneration (AMD): the protective effect against retinal pigmented epithelium damage and vision loss.

A APOE4-HFC mouse model of age-related macular degeneration (AMD) (aged human apolipoprotein E 4 targeted replacement mice on a high-fat, cholesterol-enriched diet) is used to demonstrate the effect of intracerebroventricular infusion of artificial cerebrospinal fluid on the risk of development or the progression of AMD. Consistent with a pathogenic role for Aβ, this mouse model of AMD (which combines multiple risk factors for AMD) is characterized by Aβ-containing deposits basal to the retinal pigmented epithelium (RPE), histopathologic changes in the RPE, and a deficit in scotopic electroretinographic response, which is reflective of impaired visual function.

During a stereotaxic surgery under deep anesthesia a guide cannula is implanted into the lateral ventricle. The cannula is connected to an iPRECIO^{®} implantable infusion pump implanted subcutaneously. CSF Volume & Production Rate in the Mouse are: CSF Volume = 35 µl; CSF Production rate = 18 µl/hr. Classic Alzet osmotic pumps have a maximal infusion rate of 0.25µL/h and require surgery to replace the empty pump every 4 weeks (pump type 2004). Based on these infusion rates and the CSF production rate in mice, classic osmotic pumps cannot be applied to realize an increased cerebrospinal fluid and glymphatic flow. Application of iPRECIO^{®} implantable infusion pumps, allows adaptation of the flow rate and also higher flow rates can be achieved. The iPRECIO^{®} implantable infusion pump uses a microprocessor controlled peristalsis mechanism for accurate controlled flow. Mice are housed individually after surgery. In a small set of control mice, the maximal tolerable infusion rate is established by slowly increasing the infusion rate. Subsequently, APOE4-HFC mice are bred and the effect of CSF infusion on histopathologic changes in the RPE and visual function are investigated. Besides the CSF-infusion group of APOE4-HFC mice (at max. tolerated flow rate), a control group is provided to control for the effect of the surgery and presence of a guide cannula. Visual function is monitored by analysis of b-wave electroretinograms (ERGs), a reliable measure of retinal activity and visual function. Mice are sacrificed and their eyes enucleated and processed for histologic study.

A reduction in the levels of Aβ and activated complement components in sub-RPE deposits, structural preservation of the RPE, and visual protection is observed in the group receiving CSF infusion compared to control. This demonstrates that CSF infusion is a promising prophylactic and therapeutic strategy for human AMD.

## Claims

1. A composition comprising cerebrospinal fluid (CSF) or a CSF-like composition for use in the prevention and/or treatment of a retinal disease;
- wherein the CSF-like composition is artificial CSF (aCSF), wherein aCSF comprises sodium ions at a concentration of 140-190 mM, potassium ions at a concentration of 2.5-4.5 mM, calcium ions at a concentration of 1-1.5 mM, magnesium ions at a concentration of 0.5-1.5 mM, phosphor ions at a concentration of 0.5-1.5 mM, chloride ions a concentration of 100-200 mM;
- wherein said retinal disease is a disease primarily affecting the retina; and
- wherein said prevention or treatment of retinal diseases is ensured by infusion into the intrathecal space, subarachnoid space or cerebral ventricles and wherein said retinal disease is selected from age-related macular degeneration, retinal vasculitis and retinal infective processes, commotio retinae, diabetic retinopathy, hereditary retinal dystrophies, ischemic insult of retinal neurons and macular edema.

2. The composition for use according to claim 1, wherein said infusion ensures an increased glymphatic system clearance in the retina.

3. The composition for use according to claim 1, wherein said infusion ensures an increase in CSF turnover.

4. The composition for use according to any one of claims 1 or 2, wherein said infusion ensures reduction of the trans-lamina cribrosa pressure difference (TLCPD).

5. The composition for use according to claim 4, wherein said infusion reduces the TLCPD to about 4 mmHg or less, preferably to 1 mmHg or 2 mmHg.

6. The composition for use according to claim 4 or 5, wherein said reduction of TLCPD is ensured by an increase of intracranial pressure (ICP).

7. The composition for use according to claims 1 to 6, wherein said TLCPD is reduced to 1 to 2 mm Hg.

8. The composition for use according to any one of claims 1 to 7, wherein said infusion is ensured by an implantable pump.

9. The composition for use according to claim 8, wherein said infusion is ensured by an apparatus for infusing fluid into the intrathecal space or the cerebral ventricles of a patient the apparatus comprising: an implantable pump; a reservoir for containing artificial cerebrospinal fluid; an infusion catheter having an inlet end coupled to the reservoir, and an outlet end coupled to the implantable pump; and an inflow catheter having an outlet end configured to be disposed in fluid communication with said intrathecal space or the cerebral ventricles, and an inlet end coupled to the implantable pump; and wherein the implantable pump is configured to selectively move artificial cerebrospinal fluid from the reservoir through the infusion catheter and the inflow.

## Patentansprüche

1. Zusammensetzung, umfassend Cerebrospinalflüssigkeit (CSF) oder eine CSF-artige Zusammensetzung zur Verwendung in der Prävention und/oder Behandlung einer Netzhauterkrankung;
- wobei eine CSF-artige Zusammensetzung künstliche CSF (aCSF) ist, wobei aCSF Natriumionen in einer Konzentration von 140 bis 190 mM, Kaliumionen in einer Konzentration von 2,5 bis 4,5 mM, Calciumionen in einer Konzentration von 1 bis 1,5 mM, Magnesiumionen in einer Konzentration von 0,5 bis 1,5 mM, Phosphorionen in einer Konzentration von 0,5 bis 1,5 mM, Chloridionen in einer Konzentration von 100 bis 200 mM umfasst;
- wobei die Netzhauterkrankung eine Erkrankung ist, die primär die Netzhaut beeinflusst; und
- wobei die Prävention oder Behandlung von Netzhauterkrankungen durch Infusion in den intrathekalen Raum, Subarachnoidalraum oder Hirnventrikel gewährleistet ist, und wobei die Netzhauterkrankung ausgewählt ist aus altersbedingter Makuladegeneration, retinaler Vaskulitis und infektiösen Prozessen der Retina, Commotio retinae, diabetischer Retinopathie, hereditären retinalen Dystrophien, ischämischem Insult von retinalen Neuronen und Makulaödem.

2. Zusammensetzung zur Verwendung nach Anspruch 1, wobei die Infusion eine erhöhte Clearance des glymphatischen Systems in der Netzhaut sicherstellt.

3. Zusammensetzung zur Verwendung nach Anspruch 1, wobei die Infusion einen Anstieg des CSF-Umsatzes sicherstellt.

4. Zusammensetzung zur Verwendung nach einem der Ansprüche 1 oder 2, wobei die Infusion Reduktion der translaminalen Cribrosa-Druckdifferenz (TLCPD) sicherstellt.

5. Zusammensetzung zur Verwendung nach Anspruch 4, wobei die Infusion die TLCPD auf etwa 4 mmHg oder weniger, vorzugsweise 1 mmHg oder 2 mmHg reduziert.

6. Zusammensetzung zur Verwendung nach Anspruch 4 oder 5, wobei die Reduktion von TLCPD durch eine Erhöhung des intrakraniellen Drucks (ICP) sichergestellt wird.

7. Zusammensetzung zur Verwendung nach Anspruch 1 bis 6, wobei die TLCPD auf 1 bis 2 mmHg reduziert wird.

8. Zusammensetzung zur Verwendung nach einem der Ansprüche 1 bis 7, wobei die Infusion durch eine implantierbare Pumpe sichergestellt wird.

9. Zusammensetzung zur Verwendung nach Anspruch 8, wobei die Infusion durch ein Gerät zum Infundieren von Flüssigkeit in den intrathekalen Raum oder die Hirnventrikel eines Patienten sichergestellt wird, wobei das Gerät umfasst: eine implantierbare Pumpe; ein Reservoir zum Aufnehmen von künstlicher Cerebrospinalflüssigkeit; einen Infusionskatheter mit einem Einlassende, das an das Reservoir gekoppelt ist, und einem Auslassende, das an die implantierbare Pumpe gekoppelt ist; und einen Einlaufkatheter mit einem Auslassende, das ausgestaltet ist, um in Fluidkommunikation mit dem intrathekalen Raum oder den Hirnventrikeln angeordnet zu werden, und einem Einlassende, das an die implantierbare Pumpe gekoppelt ist; und wobei die implantierbare Pumpe ausgestaltet ist, um künstliche Cerebrospinalflüssigkeit selektiv aus dem Reservoir durch den Infusionskatheter und den Einlauf zu bewegen.

## Revendications

1. Composition comprenant un fluide cérébrospinal (CSF) ou une composition de type CSF pour une utilisation dans la prévention et/ou le traitement d'une maladie rétinienne ;
- la composition de type CSF étant un CSF artificiel (aCSF), un aCSF comprenant des ions sodium à une concentration de 140 à 190 mM, des ions potassium à une concentration de 2,5 à 4,5 mM, des ions calcium à une concentration de 1 à 1,5 mM, des ions magnésium à une concentration de 0,5 à 1,5 mM, des ions phosphore à une concentration de 0,5 à 1,5 mM, des ions chlorure à une concentration de 100 à 200 mM ;
- ladite maladie rétinienne étant une maladie affectant principalement la rétine ; et
- ladite prévention ou ledit traitement de maladies rétiniennes étant assuré(e) par une perfusion dans l'espace intrathécal, l'espace subarachnoïde ou les ventricules cérébraux et ladite maladie rétinienne étant choisie parmi la dégénérescence maculaire liée à l'âge, la vascularite rétinienne et des processus infectieux rétiniens, la rétine commune, la rétinopathie diabétique, les dystrophies rétiniennes héréditaires, un accident ischémique de neurones rétiniens et un œdème maculaire.

2. Composition pour une utilisation selon la revendication 1, ladite perfusion assurant une clairance du système glymphatique augmentée dans la rétine.

3. Composition pour une utilisation selon la revendication 1, ladite perfusion assurant une augmentation du renouvellement de CSF.

4. Composition pour une utilisation selon l'une quelconque des revendications 1 ou 2, ladite perfusion assurant une réduction de la différence de pression de trans-lamina cribrosa (TLCPD).

5. Composition pour une utilisation selon la revendication 4, ladite perfusion réduisant la TLCPD jusqu'à environ 4 mmHg ou moins, préférablement de 1 mmHg ou de 2 mmHg.

6. Composition pour une utilisation selon la revendication 4 ou 5, ladite réduction de TLCPD étant assurée par une augmentation de la pression intracrânienne (ICP).

7. Composition pour une utilisation selon les revendications 1 à 6, ladite TLCPD étant réduite jusqu'à 1 à 2 mmHg.

8. Composition pour une utilisation selon l'une quelconque des revendications 1 à 7, ladite perfusion étant assurée par une pompe implantable.

9. Composition pour une utilisation selon la revendication 8, ladite perfusion étant assurée par un appareil pour la perfusion d'un fluide dans l'espace intrathécal ou les ventricules cérébraux d'un patient, l'appareil comprenant : une pompe implantable ; un réservoir pour contenir un fluide cérébrospinal artificiel ; un cathéter de perfusion possédant une extrémité d'entrée couplée au réservoir, et une extrémité de sortie couplée à la pompe implantable ; et un cathéter d'entrée possédant une extrémité de sortie configurée pour être disposée en communication fluidique avec ledit espace intrathécal ou les ventricules cérébraux, et une extrémité d'entrée couplée à la pompe implantable ; et la pompe implantable étant configurée pour déplacer sélectivement un fluide cérébrospinal artificiel du réservoir à travers le cathéter de perfusion et l'entrée.
